# EUROPEAN PATENT APPLICATION

(11) **EP 4 487 826 A1**
(43) Date of publication of application: **08.01.2025**
(21) Application number: 23763710.3
(22) Date of filing: 02.03.2023
(51) Int. Cl.: A61F 5/042, A61F 5/32, A61H 1/00, A61H 1/02, A61G 13/00

(54) **POSTURE CORRECTOR**

(30) Priority: 04.03.2022 KR 20220028237
(71) Applicant: Seo, Sang Sul, Jungnang-gu, Seoul 02178 (KR)
(72) Inventor: Seo, Sang Sul, Jungnang-gu, Seoul 02178 (KR)
(74) Representative: Abel & Imray LLP
(86) International application number: PCT/KR2023/002879
(87) International publication number: WO 2023/167512

(57) **Abstract**

The present invention relates to a posture corrector, comprising a bed on which a user can lie down to correct the spine by repeatedly lifting and lowering the head or head and shoulders at the same time, in which a chest compression device and a pubis compression device for fixing the ribcage and pubis of the user lying on the bed are combined with a fixing belt such that the user can easily compress and fix the ribcage and pubis of the user.

## Description

### Technical Field

The present disclosure relates to a posture corrector and, more particularly, to a posture corrector designed to correct the spine by repeatedly raising and lowering the head and shoulders of a user and applying pressure to the pubis and ribcage by means of a compression part while the user lies down for patients who avoid strong stimulation associated with surgery or acupuncture.

### Background Art

The spine is the most important bone structure that supports the human body. Nevertheless, contemporary people who spend a lot of time sitting down often tilt their heads forward or bend forward at the waist to read books or use computers and, in particular, test takers often spend long periods of time with their heads down to study.

If a person repeatedly adopts this bad posture, the person's head will stick out further than the person's shoulders, the person's back will relatively retreat backwards and bend, and the person's cervical spine will tend to become straight or curved forward. In addition, because the muscles behind the neck and both shoulders need to support the weight of the head to prevent the head from leaning forward, the muscles are always tight and rigid, which can lead to cervical and spinal diseases.

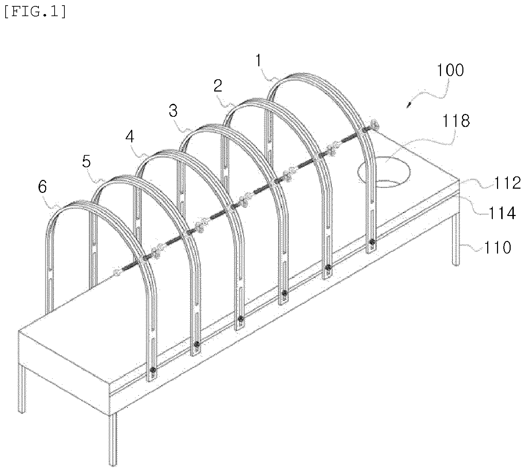

In order to solve this problem, Korean Patent Application Publication No. 10-2011-0053729 (hereinafter referred to as "cited invention") discloses, as shown in FIG. 1, a scoliosis correction device that corrects the spine by applying force at various angles and pressure while a patient with scoliosis is lying face down.

According to the cited invention, an unstable spine may be corrected by directly pressing the user's spine by means of a guide bar installed by a guide bar fixing means on a rail on which a guide rail is installed.

However, such a method may cause pain by directly pressing and stimulating the body area that needs correction, and may only serve to temporarily press the unstable body area when worn. Moreover, the device of the cited invention may cause pain by directly pressing the spine of a user who wants to effectively correct his/her posture while avoiding strong stimulation associated with surgery, procedures, and acupuncture.

### Disclosure

### Technical Problem

The present disclosure is intended to solve the above problems occurring in the related art. An objective of the present disclosure is to provide a posture corrector including a bed on which a user can lie down is equipped with a compression part to press and secure the ribcage and pubis (pubic bone) of the user to allow the user to correct the spine by repeating the movement of lifting and lowering the head or head and shoulders at the same time while lying on the bed and adjusting the degree of compression on the ribcage and pubis.

### Technical Solution

In order to achieve the above-mentioned objectives, there is provided a posture corrector including: a bed configured to support a body of a user lying thereon; and a compression part in which a fixing belt formed in a belt shape and configured to wrap around and secure the body of the user lying on the bed is combined with each of a chest compression device that holds a user's ribcage in place and a pubis compression device that fixes a user's pubis in order to press the user's ribcage and pubis.

In addition, opposite ends of the fixing belt may be coupled to at least one power device provided under the bed, and as the opposite ends of the fixing belt are pulled by the power device, the fixing belt may adjust a degree of compression of the compression part that fixes the user.

In addition, the bed may include a controller on a side thereof to control the degree of compression of the compression part delivered to the user by means of the power device.

In addition, the pubis compression device may include: a cushion whose longitudinal cross-section is curved to cover a user's abdomen and a pelvis and having a crescent-shaped indentation in an area corresponding to the user's pubis; and a pubis pressing plate that is placed in the crescent-shaped indentation area of the cushion and presses the user's pubis as the pubis pressing plate is pressed by the fixing belt.

In addition, the pubis compression device may further include an auxiliary cushion having a shape corresponding to a bottom surface of the cushion, wherein the auxiliary cushion may be provided in multiple layers and formed such that the multiple layers of an end thereof in a direction of a user's chest may be combined with each other to be able to rotate in the direction of the user's chest, and the pubis compression device may further include: a cover provided on an upper surface of the pubis compression device; and a pressing tool provided in a form of a threaded shaft and provided with a handle at a first end thereof to enable gripping, while a second end of the pressing tool is coupled to the pubis pressing plate through a hole formed in the fixing belt, wherein the pressing tool may be placed in the area where the pubis pressing plate is disposed within the pubis compression device, and as the handle rotates due to an external force, the pressing tool may be inserted downward along a pressing hole formed in the cover, and the pubis pressing plate coupled with the pressing tool may move downward along sidewalls of the cover to press the pubis of the user.

### Advantageous Effects

According to the present disclosure, a bed on which a user can lie down is provided, and a chest compression device and a pubis compression device for fixing the ribcage and pubis of the user lying on the bed are combined with a fixing belt so that the user can easily press and secure his/her ribcage and pubis.

Furthermore, the pubis compression device is equipped with a pubis pressing plate for pressing the user's pubic bone so as to effectively press the user's pubic bone, and the user can adjust the degree of pressure on the pubic bone by himself/herself by means of a handle.

Furthermore, the degree of compression on the user's ribcage and pubis can be precisely controlled through a power mechanism.

### Description of Drawings

FIG. 1 is an example of a conventional case.
FIG. 2 is a perspective view showing the front structure of a posture corrector according to an embodiment of the present disclosure.
FIG. 3 is an exemplary drawing showing the side structure of a posture corrector according to an embodiment of the present disclosure.
FIG. 4 is a plan view showing the front structure of a posture corrector according to an embodiment of the present disclosure.
FIG. 5 is an exemplary drawing showing the operation of a posture corrector according to an embodiment of the present disclosure.
FIG. 6 is an exemplary drawing showing the lower structure of a posture corrector according to an embodiment of the present disclosure.
FIG. 7 is a perspective view showing the front structure of a pubis compression device according to an embodiment of the present disclosure.
FIG. 8 is an assembly example of a pubis compression device according to an embodiment of the present disclosure.
FIG. 9 is an exemplary drawing showing a structure in which a fixing belt according to an embodiment of the present disclosure penetrates a pubis compression device.
FIG. 10 is a front view showing the front structure of a pubis compression device according to an embodiment of the present disclosure.
FIG. 11 is a side view showing the side structure of a pubis compression device according to an embodiment of the present disclosure.
FIG. 12 is an exemplary drawing showing the operation of a pubis compression device according to an embodiment of the present disclosure.

### Mode for Invention

In the present disclosure, proposed is a posture corrector including a bed on which a user can lie down is provided, and a chest compression device and a pubis compression device for fixing the ribcage and pubis of the user lying on the bed are combined with a fixing belt so that the user can easily press and secure his/her ribcage and pubis.

The scope of the present disclosure is not limited to the embodiments described below, and various modifications may be made by a person having ordinary knowledge in the relevant technical field without departing from the technical spirit of the present disclosure.

Hereinafter, a posture corrector A of the present disclosure will be described in detail with reference to the attached drawings 2 to 12.

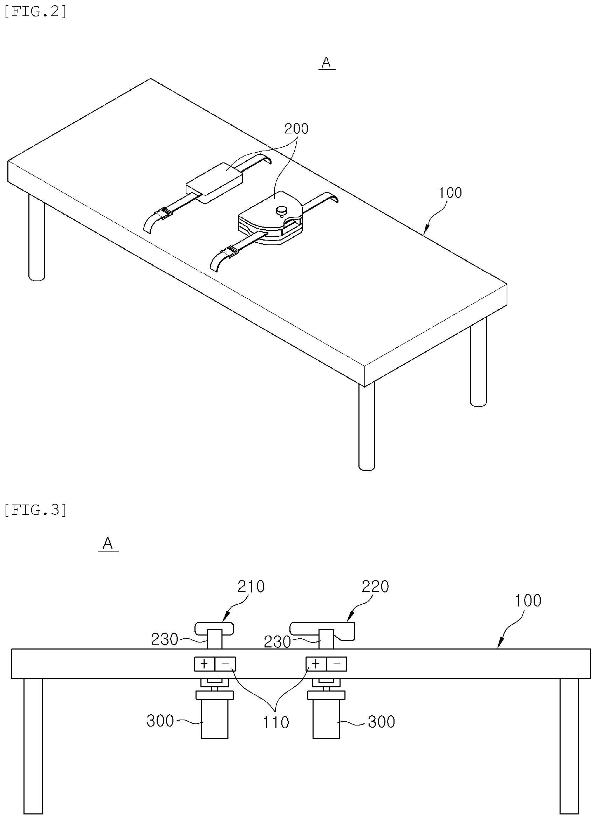

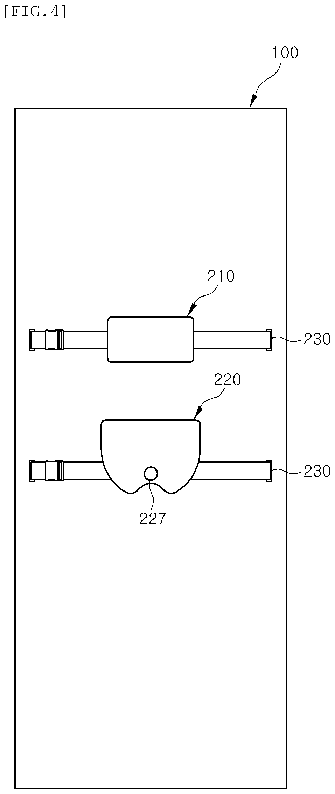

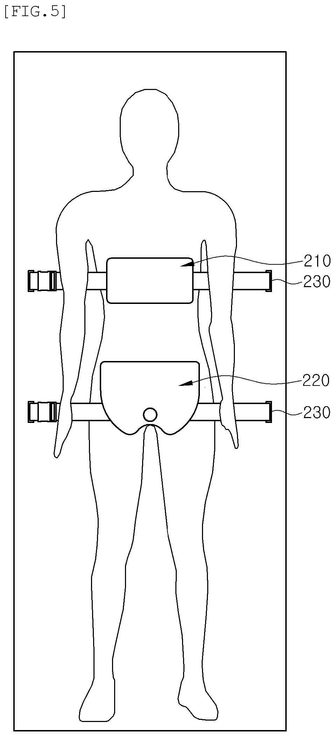

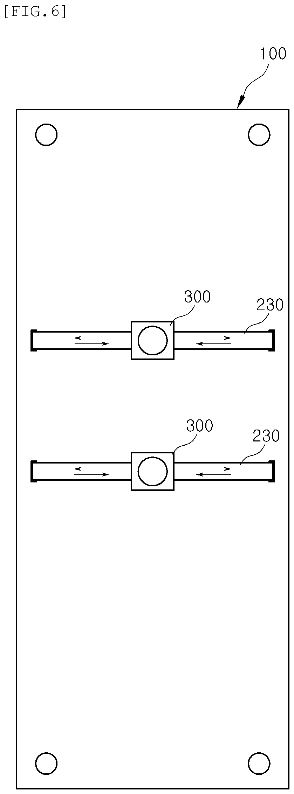

As shown in FIGS. 2 to 6, the posture corrector A of the present disclosure may include: a bed 100 that is formed to be spaced a certain distance from the ground and allows a user to lie thereon and support the body; and a compression part 200 in which a fixing belt 230 that is formed in a belt shape, and wraps around and secures the body of the user lying on the bed 100 is combined with each of a chest compression device 210 that holds the user's ribcage in place and a pubis compression device 220 that fixes the user's pubic bone.

As shown in FIGS. 2 to 3, the bed 100 may be provided with a plurality of support portions (not shown) so as to be spaced apart from the ground at a certain distance. At least two pairs of belt through-holes, each pair corresponding to the width of the fixing belt 230, may be provided inside the bed 100, and a power device 300 located on the same line as each pair of belt through-holes may be provided on the bottom of the bed 100.

In this case, the fixing belt 230, which is coupled to each of the chest compression device 210 and the pubis compression device 220, may be fastened to each pair of the at least two pairs of belt through-holes.

As shown in FIGS. 3 to 6, both ends of the fixing belt 230 230 passing through each pair of the at least two pairs of belt through-holes may be coupled to the power device 300 provided on the bottom of the bed 100, and as the power device 300 operates, both ends of the fixing belt 230 are pulled by the power device 300 to adjust the degree of compression of the compression part 200 that secures the user lying on the bed 100.

At this time, the fixing belt 230 has a coupling member provided at one point thereof so that the user may easily attach and detach the fixing belt.

In addition, as shown in FIGS. 2 to 3, the bed 100 may be provided with a controller 110 configured to control the operation of the power device 300 in an area that can be reached by the user lying on the bed 100. In this case, as shown in FIGS. 2 to 3, the controller 110 may be provided in the form of a "+" and "-" button, but may also be provided in various forms such as characters, notations, or marks that can be identified by the user.

In addition, in order to adjust the degree of compression for each of the chest compression device 210 and the pubis compression device 220, the controller 110 may be provided on one side adjacent to the chest compression device 210 and on one side adjacent to the pubis compression device 220.

For example, when the user presses "+" button of the controller 110, the degree of compression of the compression part 200 may increase, and when the user presses "-" button of the controller 110, the degree of compression of the compression part 200 may decrease.

As shown in FIGS. 2 to 6, the compression part 200 may include: the chest compression device 210 that is made of a soft material as the chest compression device 210 touches the user's ribcage and has one fixing belt 230 penetrating therethrough to surround and press the user's ribcage; the pubis compression device 220 including the cushion 224 whose longitudinal cross-section is curved to cover the user's abdomen and pelvis and having a crescent-shaped indentation in the area corresponding to the user's pubis, and a pubis pressing plate 228 that is placed in the crescent-shaped indentation area of the cushion 224 and presses the user's pubis as the pubis pressing plate 228 is pressed by the other fixing belt 230 penetrating the cushion 224.

As shown in FIGS. 2 to 6, the chest compression device 210 may be sized to cover the user's ribcage and may have a width smaller than the width of the user's body. Additionally, the part that comes into contact with the user's body may be made of a soft material.

In this case, the chest compression device 210 may have a longitudinal cross-section formed in a shape corresponding to the user's chest, and one side and the other side of the chest compression device 210 may be penetrated to have a hole and the fixing belt 230 is inserted along the hole through the one side and the other side of the chest compression device 210 to surround the user's ribcage, so that the user's ribcage may be compressed and fixed at the same time.

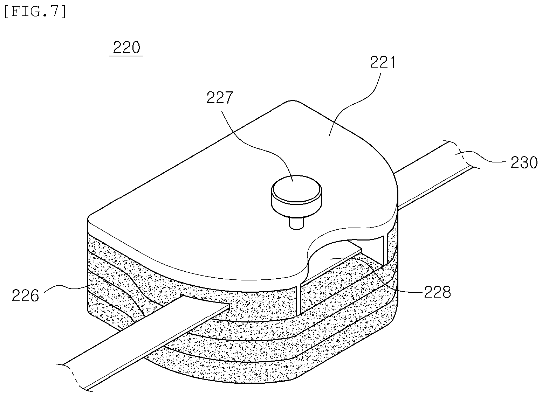

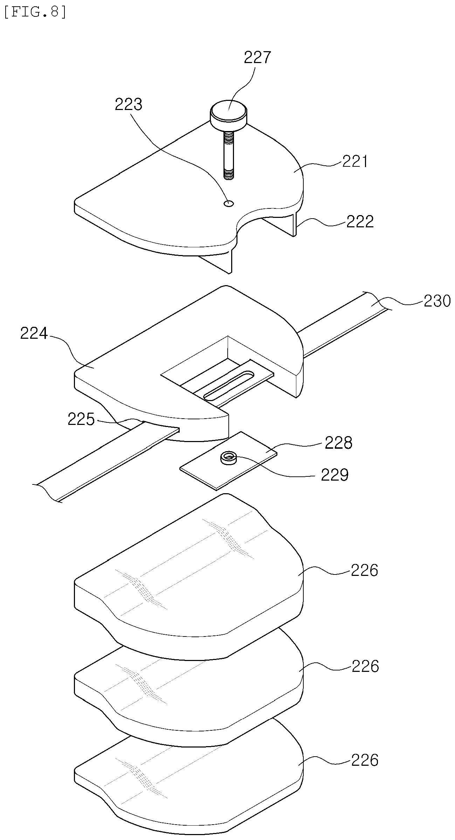

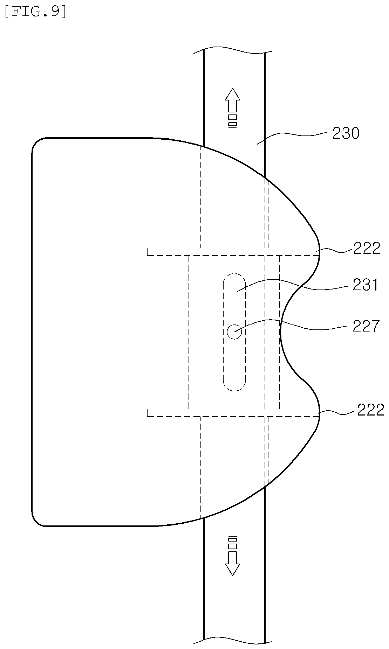

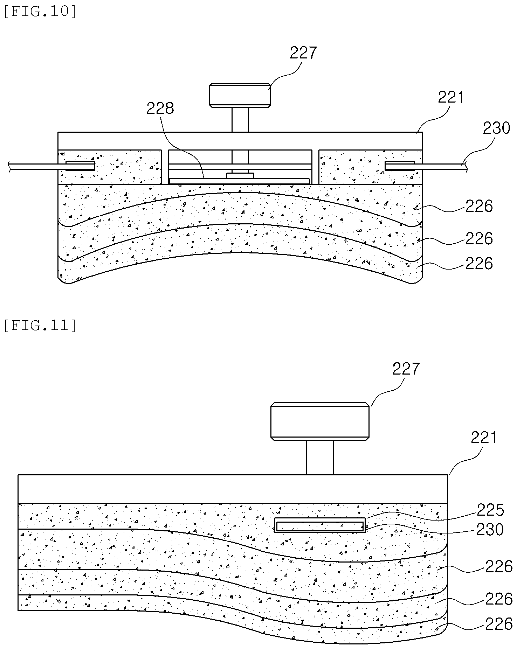

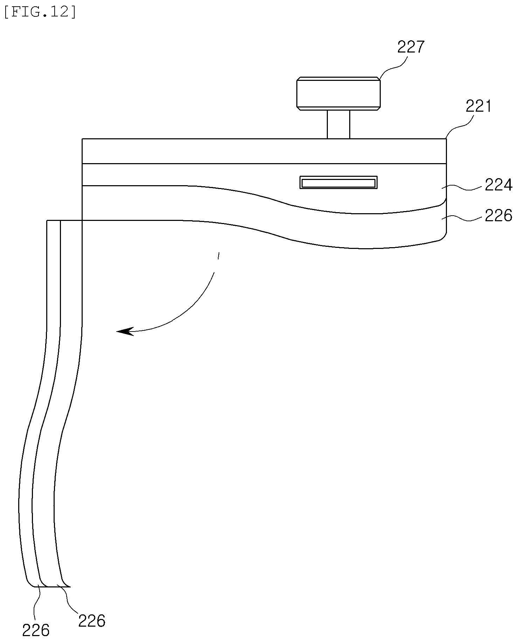

As shown in FIGS. 7 to 12, the pubis compression device 220 may include: a cover 221 provided on the upper surface of the pubis compression device 220 and made of a material with a certain strength; and the cushion 224 provided below the cover 221, having a longitudinal cross-section formed in a curved shape, and having a transverse cross-section formed to be lower in height along the curve of the user's abdomen toward the user's chest, so as to cover the user's abdomen and pubis and have a crescent-shaped indentation in the area corresponding to the user's pubis.

In addition, the pubis compression device 220 may further include an auxiliary cushion 226 having a shape corresponding to the bottom surface of the cushion 224. The auxiliary cushion 226 may be formed in multiple layers, and may be formed such that the multiple layers of one end thereof in the direction of the user's chest are combined with each other to be able to rotate in the direction of the user's chest. Due to this, the height of the pubis compression device 220 may be adjusted, and the pubis compression device 220 may be made to cover the user's upper abdomen.

In addition, the pubis compression device 220 may further include a pressing tool 227 penetrating the cover 221, and pressing the pubis pressing plate 228 downward as the pressing tool 227 is coupled to the pubis pressing plate 228 disposed in the indented area of the cushion 224 and a handle of the pressing tool 227 is rotated.

As shown in FIGS. 7 to 8, the cover 221 may have a cross-section shaped like a human pelvis, and may be provided with a plurality of sidewalls 222 on the lower surface thereof facing each other and having a predetermined height downward. The cover may have a pressing hole 223 formed at a point between the sidewalls 222, so that the pressing tool 227 may penetrate the cover 221 and press downward.

The sidewall 222 provided on the cover 221 may be formed in a shape corresponding to the recessed area formed in the cushion 224 and may be fitted to come into contact with the wall surface of the indented area in the cushion 224. In addition, a space may be formed as the open upper side of the crescent-shaped indentation area of the cushion 224 is closed by the cover 221.

As shown in FIGS. 7 to 8, the pressing tool 227 may be provided in the form of a threaded shaft and provided with the handle at one end thereof to enable gripping. The other end of the pressing tool 227 may be inserted through the pressing hole 223 of the cover 221 and coupled to the pubis pressing plate 228 disposed in the crescent-shaped indentation area of the cushion 224.

In this case, as the cover 221 and the sidewalls 222 are combined, the pubis pressing plate 228 may move up and down along the sidewalls 222 of the cover 221 while being formed in the shape of a pubic bone in the space created by the sidewalls 222 of the cover 221 and the indented area of the 224, and may have a cylindrical pressing tool fixing pillar 229 on the upper surface thereof so as to be coupled to the other end of the pressing tool 227.

In addition, the pubis compression device 220 may have a cushion penetration hole 225 penetrating to a size corresponding to the width of the fixing belt 230 on each side of the crescent-shaped indentation area where the sidewalls 222 of the cover 221 are inserted, so that the fixing belt 230 may penetrate opposite sides of the pubis compression device 220. The fixing belt 230 may have a rectangular fixing belt hole 231 formed at a position corresponding to the pressing tool fixing pillar 229 of the pubis pressing plate 228 so as to press the remaining area of the pubis pressing plate 228 except for the pressing tool fixing pillar 229.

In this case, as the handle rotates due to an external force, the pressing tool 227 is inserted downward along the pressing hole 223 formed in the cover 221, and the pubis pressing plate 228 coupled with the pressing tool 227 moves downward along the sidewalls 222 of the cover 221 to press the pubis of the user.

### Sequence Listing Free Text

[Description of Reference Numerals]
A: posture corrector
100: bed 110: controller
200: compression part
210: chest compression device 220: pubis compression device
221: cover 222: sidewall
223: pressing hole 224: cushion
225: cushion penetration hole 226: auxiliary cushion
227: pressing tool 228: pubis pressing plate
229: pressing tool fixing pillar
230: fixing belt 231: fixing belt hole
300: power device

## Claims

1. A posture corrector, comprising:
a bed (100) configured to support a body of a user lying thereon; and
a compression part (200) in which a fixing belt (230) formed in a belt shape and configured to wrap around and secure the body of the user lying on the bed (100) is combined with each of a chest compression device (210) that holds a user's ribcage in place and a pubis compression device (220) that fixes a user's pubis in order to press the user's ribcage and pubis,
wherein the pubis compression device (220) comprises:
a cushion (224) whose longitudinal cross-section is curved to cover a user's abdomen and a pelvis and having a crescent-shaped indentation in an area corresponding to the user's pubis; and
a pubis pressing plate (228) that is placed in the crescent-shaped indentation area of the cushion (224) and presses the user's pubis as the pubis pressing plate (228) is pressed by the fixing belt (230).

2. The posture corrector of claim 1, wherein opposite ends of the fixing belt (230) are coupled to at least one power device (300) provided under the bed (100), and as the opposite ends of the fixing belt (230) are pulled by the power device (300), the fixing belt (230) adjusts a degree of compression of the compression part (200) that fixes the user.

3. The posture corrector of claim 2, wherein the bed (100) comprises a controller (110) on a side thereof to control the degree of compression of the compression part (200) delivered to the user by means of the power device (300).

4. The posture corrector of claim 1, wherein the pubis compression device (220) further comprises:
an auxiliary cushion (226) having a shape corresponding to a bottom surface of the cushion (224),
wherein the auxiliary cushion (226) is provided in multiple layers and formed such that the multiple layers of an end thereof in a direction of a user's chest are combined with each other to be able to rotate in the direction of the user's chest, and
the pubis compression device (220) further comprises:
a cover (221) provided on an upper surface of the pubis compression device (220); and
a pressing tool (227) provided in a form of a threaded shaft and provided with a handle at a first end thereof to enable gripping, while a second end of the pressing tool (227) is coupled to the pubis pressing plate (228) through a hole formed in the fixing belt,
wherein the pressing tool (227) is placed in the area where the pubis pressing plate (228) is disposed within the pubis compression device (220), and as the handle rotates due to an external force, the pressing tool (227) is inserted downward along a pressing hole (223) formed in the cover (221), and the pubis pressing plate (228) coupled with the pressing tool (227) moves downward along sidewalls (222) of the cover (221) to press the pubis of the user.
